(19) Europäisches Patentamt
European Patent Office
Office européen des brevets

(11) EP 3 562 465 B2

(12) **NEW EUROPEAN PATENT SPECIFICATION**
After opposition procedure

(45) Date of publication and mention of the opposition decision:
**06.03.2024 Bulletin 2024/10**

(45) Mention of the grant of the patent:
**31.03.2021 Bulletin 2021/13**

(21) Application number: **17829537.4**

(22) Date of filing: **21.12.2017**

(51) International Patent Classification (IPC):
***A61K 8/34*** (2006.01) ***A61K 8/39*** (2006.01)
***A61K 8/49*** (2006.01) ***A61Q 19/00*** (2006.01)
***A61K 47/08*** (2006.01) ***A61K 47/22*** (2006.01)

(52) Cooperative Patent Classification (CPC):
**A61K 8/345; A61K 8/39; A61K 8/498; A61Q 19/00;**
A61K 47/08; A61K 47/22

(86) International application number:
**PCT/US2017/067780**

(87) International publication number:
**WO 2018/125734 (05.07.2018 Gazette 2018/27)**

(54) **NOVEL ANTIOXIDANTS FOR COSMETICS AND PHARMACEUTICAL COMPOSITIONS CONTAINING GLYCEROL ALKYL ETHERS**

NEUARTIGE ANTIOXIDANSMITTEL FÜR KOSMETISCHE UND PHARMAZEUTISCHE ZUSAMMENSETZUNGEN MIT GLYCEROLALKYLETHERN

NOUVEAUX ANTIOXYDANTS POUR COSMÉTIQUES ET COMPOSITIONS PHARMACEUTIQUES CONTENANT DES ÉTHERS ALKYLIQUES DE GLYCÉROL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.12.2016 US 201662439954 P**
**24.03.2017 US 201762475977 P**

(43) Date of publication of application:
**06.11.2019 Bulletin 2019/45**

(73) Proprietor: **Sachem, Inc.**
**Austin, TX 78704 (US)**

(72) Inventor: **ENGEL, Tim**
**5037 DR Tilburg (NL)**

(74) Representative: **Scholz, Volker**
**Boehmert & Boehmert**
**Anwaltspartnerschaft mbB**
**Pettenkoferstrasse 22**
**80336 München (DE)**

(56) References cited:
**EP-A1- 1 537 849**
**WO-A1-01/93825**
**WO-A2-2009/101262**
**CN-A- 105 997 754**
**DE-A1- 10 063 345**
**DE-A1-102006 062 566**
**FR-A1- 2 923 159**
**US-A1- 2009 130 029**
**US-B2- 6 956 062**

- **Anonymous: "Skin Soothing Hydrating Lotion - Record ID: 3635541", GNPD- MINTEL GNPD- MINTEL, 31 December 2015 (2015-12-31), pages 1-4, Retrieved from the Internet: URL:https://www.gnpd.com/sinatra/recordpage/3635541/from_search/ZdUXdYhdS0/?page=1 [retrieved on 2022-02-23]**
- **Anonymous: "Charcoal Rescue Masque- Record ID: 3689747", GNPD- MINTEL GNPD- MINTEL, 31 January 2016 (2016-01-31), pages 1-4, Retrieved from the Internet: URL:https://www.gnpd.com/sinatra/recordpage/3689747/from_search/Tz5Mk1W4HA/?page=1 [retrieved on 2022-02-23]**
- **Anonymous: "Cream - Record ID: 2842097", GNPD- MINTEL GNPD- MINTEL, 31 January 2015 (2015-01-31), pages 1-4, Retrieved from the Internet: URL:https://www.gnpd.com/sinatra/recordpage/2842097/from_search/WS0M5I0Od1/?page=1 [retrieved on 2022-02-23]**
- **Anonymous: "Dark Spot Corrector - Record ID: 4173965", GNPD - MINTEL GNPD - MINTEL, 31 July 2016 (2016-07-31), pages 1-3, Retrieved from the Internet: URL:https://www.gnpd.com/sinatra/recordpage/4173965/from_search/sj8e124a64/?page=1 [retrieved on 2022-02-23]**

- Anonymous: "Body Milk - Record ID: 1714537", GNPD- MINTEL GNPD- MINTEL, 31 January 2012 (2012-01-31), pages 1-3, Retrieved from the Internet: URL:https://www.gnpd.com/sinatra/recordpage/1714537/from_search/BSgTEuJsjz/?page=1 [retrieved on 2022-02-23]
- Anonymous: "Milk - Record ID: 2104385", GNPD- MINTEL GNPD- MINTEL, 31 July 2013 (2013-07-31), pages 1-5, Retrieved from the Internet: URL:https://www.gnpd.com/sinatra/recordpage/2104385/from_search/KMF8Kz0zKa/?page=1 [retrieved on 2022-02-23]
- Anonymous: "Mousse Glow- Record ID: 2561447", MINTEL MINTEL, 31 July 2014 (2014-07-31), pages 1-3, Retrieved from the Internet: URL:https://www.gnpd.com/sinatra/recordpage/2561447/from_search/mjTeddzJ3J/?page=1 [retrieved on 2022-02-23]
- Anonymous: "Hydrating Mousse SPF 15- Record ID: 2322288", MINTEL MINTEL, 28 February 2014 (2014-02-28), pages 1-3, Retrieved from the Internet: URL:https://www.gnpd.com/sinatra/recordpage/2322288/from_search/LRJZWLibNp/?page=1 [retrieved on 2022-02-23]
- Anonymous: "Oil-Free All Dav AcneControl Leave-On Foam- Record ID: 1762961", MINTEL, 30 April 2012 (2012-04-30), pages 1-3,
- Anonymous: "Ethylhexylglycerin", Personalcarecouncil.org Personalcarecouncil.org, 2 December 2021 (2021-12-02), pages 1-1, Retrieved from the Internet: URL:https://online.personalcarecouncil.org/jsp/IngredientDetail.jsp?monoid=6765 [retrieved on 2022-02-23]

## Description

TECHNICAL FIELD

The present invention relates to compositions comprising glycerol monoalkyl ethers for use in cosmetic and pharmaceutical preparations and in technical products, and further comprising an anti-oxidant.

BACKGROUND

Glycerol monoalkyl ethers are used as additives for cosmetic and pharmaceutical preparations. Glycerol monoalkyl ethers are used as physiologically compatible organic solvents. For example, 3-[(2-ethylhexyl)oxy]-1,2-propanediol has been used for some years as a deodorant active ingredient and skin care additive in cosmetic and pharmaceutical preparations. In such products, the glycerol monoalkyl ethers are added to the products in the form of a concentrate or, occasionally, as a pure glycerol monoalkyl ether.

During manufacture, storage and use, the glycerol monoalkyl ether, its concentrate and its dilute solution, which may be referred to as a working solution, are subject to stringent requirements for stability when used in personal care products, such as cosmetics, and pharmaceutical preparations. Because glycerol monoalkyl ethers occur naturally, both the naturally produced and the synthetically prepared members of the class of substance have become desirable for use in personal care products, such as cosmetics, and pharmaceutical preparations, and are widely accepted by manufacturers of cosmetics and pharmaceuticals as well as end users. It is known that glycerol monoalkyl ethers are subject to oxidative degradation, and so may be provided from the manufacturer with an antioxidant additive to inhibit such degradation. Over time, signs of instability, including formation of peroxide and formaldehyde, have been observed when the glycerol monoalkyl ethers are not stored properly.

Formation of peroxides and formaldehyde in cosmetic and pharmaceutical preparations can cause a number of problems. Formaldehyde is, in and of itself, highly undesirable in any exposure to humans or animals. In skin care products, the presence of peroxides can cause skin problems such as dermatosis. Peroxides can cause changes in the odor of stored products, due to oxidation of natural fats and oils present in such formulations. Peroxides may result in color changes, particularly in oil-in-water emulsions containing glycerol monoalkyl ethers. Peroxides can result in the formation of low molecular weight decomposition products that can be detected by chemical analysis, and in some cases, by smell. These problems resulting from peroxide formation in compositions containing glycerol monoalkyl ethers can result in rejection of products by quality control, or, worse, by customers and end users.

U.S. Patent No. 6,956,062 discloses a number of antioxidants for use with glycerol monoalkyl ethers, including Vitamin E.

A drawback to using the antioxidants disclosed in U.S. Patent No. 6,956,062 is that such antioxidants, as natural products, can be difficult to purify. Disadvantages of vitamin E may include the fact that sources and identity are not always clear, for example, whether the vitamin E material is synthetic or natural, the possibility that impurities, including unwanted color may be introduced, and the possibility that use of vitamin E may result in hypervitaminosis E from too much vitamin E being exposed to a user's body via a cosmetic or pharmaceutical composition containing same.

Therefore, a continuing need exists for new and improved antioxidants for use with glycerol monoalkyl ethers, particularly for use in personal care products, such as cosmetics, and pharmaceutical preparations.

CN 105997754 A provides a preparation method of camellia extraction liquid. The method comprises the steps of 1) removing impurities of fresh camellia and/or fresh camellia buds; cutting up the fresh camellia and/or fresh camellia buds into small sections for use as raw materials; 2) taking a certain number of raw materials; putting the raw materials into an ultrasonic kettle; adding a solvent with a mass ratio being 1:(3-30); uniformly stirring the mixture; adding a certain amount of inorganic acid and/or organic acid; regulating the pH value to 1.3 to 3.0; soaking the materials for 10 to 30 minutes; 3) performing ultrasonic treatment for 30 to 60 minutes; controlling the ultrasonic frequency to be 20 to 25 kHz; then performing filtration, sterilization and detection to obtain camellia extraction liquid.

DE 10 2006 062 566 A1 discloses topical cosmetic and dermatological compositions which contain, in a suitable cosmetic or dermatological carrier, a combination of at least one $\alpha$-monoalkyl glycerol ether and at least one of 1,2-octanediol and 1,2-hexanediol.

FR 2923159 A1 discloses the cosmetic use of at least one glycerol monoalkyl ether, in a composition comprising a medium, as antioxidant or anti radical agent.

EP 1 537 849 A1 disclose a cosmetic or dermatological composition comprising licochalcone A or a licorice extract and a soya bean extract or isoflavonoide derived from a soya bean extract.

DE 19063345 A1 discloses light protective cosmetic or dermatological emulsions which are characterized in that they contain (a) one or more partially neutralized esters of monoglycerides and/or diglycerides of saturated fatty acids with acetic acid and (b) at least one benzotriazole derivative.

The present inventors have discovered that a new class of antioxidant compounds provide particularly excellent

results in preventing or at least reducing the incidence of the foregoing problems that may be encountered from the use of glycerol monoalkyl ethers in products such as cosmetics and pharmaceuticals.

Thus, in one embodiment, the present invention relates to a composition comprising:

a. a glycerol alkyl ether of the general formula (Ia) or (Ib):

$$R\text{-}O\text{-}CH_2\text{-}CHOH\text{-}CH_2OH \qquad (Ia)$$

or

$$H\text{-}O\text{-}CH_2\text{-}CH(OR)\text{-}CH_2OH \qquad (Ib)$$

wherein, in general formula (Ia) or (Ib), R is a $C_3$-$C_{18}$ alkyl or alkenyl group, in which the alkyl or alkenyl group is branched or unbranched, unsubstituted or substituted by one or more hydroxyl, one or more $C_1$-$C_4$ alkoxy groups, or both one or more hydroxyl and one or more $C_1$-$C_4$ alkoxy groups, and the $C_3$-$C_{18}$ alkyl group, whether branched or unbranched, unsubstituted or substituted, is optionally interrupted by up to four oxygen atoms, or R is a $C_6$-$C_{10}$ aromatic hydrocarbon, unsubstituted or substituted by one or more hydroxyl, one or more $C_1$-$C_4$ alkoxy groups, or both one or more hydroxyl and one or more $C_1$-$C_4$ alkoxy groups, and

b. one or more compound selected from naringenin and eriodyctiol,

wherein the composition contains from 300 ppm to 900 ppm of the one or more compound selected from naringenin and eriodyctiol, based on the content of the glycerol alkyl ether.

The present invention also provides a cosmetic of pharmaceutical composition according to claim 9.

The foregoing compositions containing compounds of general formulae (Ia) or (Ib) and one or more compound selected from naringenin and eriodyctiol, and the method of preserving the compounds of general formula (Ia) or (Ib) for use in a cosmetic or pharmaceutical formulation, provides a novel and unexpectedly excellent method of providing a stable product containing the glycerol monoalkyl ether of general formula (Ia) or (Ib). The present invention thereby provides a solution to the long-standing problem of providing such a versatile and stable composition, and to the long-felt need for compositions that are particularly stable.

BRIEF DESCRIPTION OF THE DRAWINGS

FIG. 1 is a graph showing stability test results comparing peroxide formation from both comparative and invention test samples.

FIG. 2 is a graph showing stability test results comparing formaldehyde formation from both comparative and invention test samples.

FIG. 3 is a graph showing the loss of 3-[(2-ethylhexyl)oxy]-1,2-propanediol on storage at elevated temperature with compounds in accordance with the present invention.

FIG. 4 is a graph showing the increase in volatile degradation products formed on storage of 3-[(2-ethylhexyl)oxy]-1,2-propanediol at elevated temperatures with compounds in accordance with the present invention.

FIG. 5 shows the results of stability testing of a blend of 3-[(n-octyl)oxy]-1,2-propanediol with a compound having general formula (II) in accordance with the present invention. FIG. 5a shows active assay and FIG. 5b shows degradation products.

FIG. 6 shows the results obtained when samples of SASKINE50™ are combined with a compound having general formula (II) in accordance with the present invention. FIG. 6a shows active assay and FIG. 6b shows degradation products.

FIG. 7 shows the minimum inhibitory concentrations for two of the glyceryl ethers with and without naringenin in accordance with the present invention.

FIG. 8 shows the results of skin irritation tests for 3-[(2-ethylhexyl)oxy]-1,2-propanediol (SASKINE50™) with and

without naringenin in accordance with the present invention.

DETAILED DESCRIPTION

As disclosed in the foregoing summary, the present invention relates to a composition, comprising

a. a glycerol alkyl ether of the general formula (Ia) or (Ib):

$R\text{-}O\text{-}CH_2\text{-}CHOH\text{-}CH_2OH$ (Ia)

or

$H\text{-}O\text{-}CH_2\text{-}CH(OR)\text{-}CH_2OH$ (Ib)

wherein, in general formula (Ia) or (Ib), R is a $C_3$-$C_{18}$ alkyl or alkenyl group, in which the alkyl or alkenyl group is branched or unbranched, unsubstituted or substituted by one or more hydroxyl, one or more $C_1$-$C_4$ alkoxy groups, or both one or more hydroxyl and one or more $C_1$-$C_4$ alkoxy groups, and the $C_3$-$C_{18}$ alkyl group, whether branched or unbranched, unsubstituted or substituted, is optionally interrupted by up to four oxygen atoms, or R is a $C_6$-$C_{10}$ aromatic hydrocarbon, unsubstituted or substituted by one or more hydroxyl, one or more $C_1$-$C_4$ alkoxy groups, or both one or more hydroxyl and one or more $C_1$-$C_4$ alkoxy groups, and
b. one or more compound selected from naringenin and eriodyctiol,

wherein the composition contains from 300 ppm to 900 ppm of the one or more compound selected from naringenin and eriodyctiol, based on the content of the glycerol alkyl ether.

It is noted that in the compounds according to general formula (Ia) or (Ib), the 2-position carbon atom of the glycerol moiety is a non-symmetric carbon atom. Accordingly, the glycerol monoalkyl ethers according to the invention can be present as racemic mixture (D,L) or in the form of enantiomer-enriched mixtures of the D- or L-form, or in the form of the pure D-enantiomer or the pure L-enantiomer. As used herein, unless specifically directed to one enantiomer or the other, there is no differentiation between the D- or L-enantiomers.

In one embodiment, the alkyl group in the glycerol monoalkyl ether is a $C_3$-$C_{12}$ alkyl group, which may be branched or unbranched. In one embodiment, the alkyl in the glycerol monoalkyl ether group is a $C_4$-$C_{12}$ alkyl group, branched or unbranched. In one embodiment, the alkyl group in the glycerol monoalkyl ether is a $C_6$-$C_{10}$ alkyl group, branched or unbranched. In one embodiment, the alkyl group in the glycerol monoalkyl ether is a $C_8$ alkyl group, branched or unbranched. In one embodiment, the $C_8$ alkyl group is n-octyl; in one embodiment, the $C_8$ alkyl group is 2-octyl; and in one embodiment, the $C_8$ alkyl group is 2-ethylhexyl.

In one embodiment, the alkyl chain in the glycerol monoalkyl ether, whether branched or unbranched, is interrupted by up to 4 oxygen atoms. Thus, the alkyl chain in the alkyl group R of the glycerol monoalkyl ether can contain alkyleneoxy groups, such as, for example, ethyleneoxy and/or propyleneoxy groups. In this embodiment, the ether-containing moiety may be obtained, for example, by the reaction of an alcohol or a diol with ethylene oxide and/or propylene oxide, as appropriate depending on the length of the ether-containing moiety and the number of interrupting oxygen atoms. In another embodiment, the ether containing moiety may be obtained by hydrolysis of an alkyl glycidyl ether. in which the alkyl group may be branched or unbranched. Suitable ether-containing moieties for the R group include $CH_3CH_2(OCH_2CH_2)_n$-, in which n = 1-5, $CH_3CH_2(OCH(CH_3)CH_2)_n$-, in which n = 1-3, for example.

In one embodiment, the R alkyl moiety contains 4 to 12 carbon atoms, in one embodiment, from 6 to 10 carbon atoms, and in one embodiment, 8 carbon atoms. In one embodiment, the 8 carbon atom alkyl group is a 2-ethylhexyl group, and in another embodiment, the 8 carbon atom alkyl group is n-octyl. In one embodiment, glycerol monoalkyl ether is 3-[(2-ethylhexyl)oxyl-1,2-propanediol, which is marketed under the trade name SASKINE50™ by SACHEM, Inc., Austin, Texas.

In one embodiment, the branched or unbranched alkyl group includes hydroxyl or alkoxyl substitution along the length of the alkyl group. When the substitution is alkoxy, the alkyl portion of the alkoxy group is a $C_1$-$C_4$ alkyl moiety. When the substitution is alkoxy, each such substituent independently may be $-OCH_3$, $-OCH_2CH_3$, $-OCH_2CH_2CH_3$, $-OCH(CH_3)_2$, $-OCH_2CH_2CH_2CH_3$, $-OCH_2CH(CH_3)_2$, $-OCH(CH_3)CH_2CH_3$ or $-OC(CH_3)_3$. There may be multiple alkoxy groups.

In one embodiment, the branched or unbranched alkyl group includes alkyl hydroxyl substitution, in which the alkyl hydroxyl includes a $C_1$-$C_4$ alkyl moiety. When the substitution is alkyl hydroxyl, each such substituent independently may be $-CH_2OH$, $-CH_2CH_2OH$, $-CH_2CH_2CH_2OH$, $-CH(CH_3)CH_2OH$, $-CH_2CH_2CH_2CH_2OH$, $-CH(CH_3)CH_2CH_2OH$, $-CH_2CH(CH_3)CH_2OH$, or $-CH_2C(CH_3)_2OH$. There may be multiple alkyl hydroxyl groups.

In one embodiment of the composition of the present invention, in the general formula (Ia) or (Ib), R is a branched

or unbranched $C_4$-$C_{12}$ alkyl group. In one embodiment, in the general formula (Ia) or (Ib), R is a branched or unbranched $C_6$-$C_{10}$ alkyl group. In one embodiment, in the general formula (Ia) or (Ib), R is a branched or unbranched $C_8$ alkyl group.

In one embodiment of the composition of the present invention, in the general formula (Ia) or (Ib), R is 2-ethylhexyl.

In one embodiment of the composition of the present invention, in the general formula (Ia) or (Ib), R is n-octyl.

In one embodiment of the composition of the present invention, in the general formula (Ia) or (Ib), R is 2-octyl.

It is noted that, in the foregoing compounds selected from naringenin and eriodyctiol, in the pyran ring, the carbon atom bonded to the phenyl ring is asymmetric Naringenin and eriodyctiol have the general formulas as shown here:

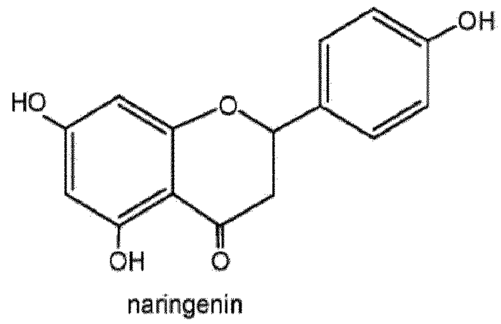

naringenin

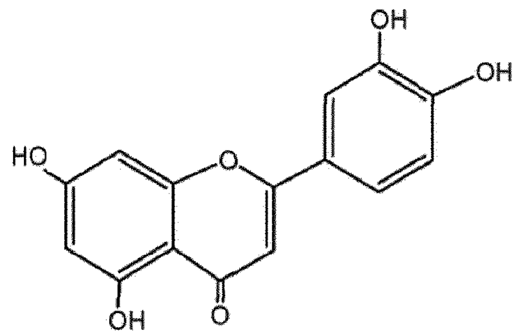

eriodictyol

Specific combinations in accordance with select embodiments of the present invention include, but are not limited to:

3-[(2-ethylhexyl)oxy]-1,2-propanediol and naringenin;

3-[(2-ethylhexyl)oxy]-1,2-propanediol and eriodyctiol;

3-[(n-octyl)oxy]-1,2-propanediol and naringenin;

3-[(n-octyl)oxy]-1,2-propanediol and eriodyctiol;

3-[(2-octyl)oxy]-1,2-propanediol and naringenin;

3-[(2-octyl)oxy]-1,2-propanediol and eriodyctiol.

When a composition in accordance with embodiments of the present invention is used in a cosmetic or pharmaceutical formulation, it will be added and the foregoing ratio will be maintained, between the compound of general formula (Ia) or (Ib) and the compound selected from naringenin and eriodyctiol. Optionally, it may be desirable to add additional quantities of the compound selected from naringenin and eriodyctiol to the cosmetic or pharmaceutical formulation, as additional antioxidant for the cosmetic or pharmaceutical composition.

In one embodiment of the composition of the present invention, the composition is a concentrate containing from 0.01 wt% to 5 wt% of the of the compound selected from naringenin and eriodyctiol, based on the total weight of the composition, with the remainder of the concentrate being one or more compound of the general formula (Ia) or (Ib).

In one embodiment, the concentrate may also include other ingredients, and the concentrate will still contain from 0.01 wt% to 5 wt% of the compound selected from naringenin and eriodyctiol, based on the total weight of the compound of general formula (Ia) or (Ib) present in the concentrate.

In one embodiment of the composition of the present invention, the compound of general formula (Ia) or (Ib) is provided to the composition at a purity of at least 99.99%, prior to addition of the compound selected from naringenin and eriodyctiol. In one embodiment, the compound of general formula (Ia) or (Ib) is provided to the composition at a purity of at least 99%, prior to addition of the compound selected from naringenin and eriodyctiol. In one embodiment, the compound of general formula (Ia) or (Ib) is provided to the composition at a purity of at least 98%, prior to addition of the compound selected from naringenin and eriodyctiol. In one embodiment, the compound of general formula (Ia) or (Ib) is provided to the composition at a purity of at least 94%, prior to addition of the compound selected from naringenin and eriodyctiol. These levels of purity of the compound of general formula (Ia) or (Ib) ensure that the product contains few if any impurities that might be absorbed through the skin of a person using a cosmetic or pharmaceutical composition made using the composition of the present invention.

In one embodiment, the composition according to the invention can be provided in the form of a working solution. A working solution may comprise from 10% by weight to about 60% by weight of the composition, including both the one or more compound according to general formula (Ia) or (Ib) and the compound selected from naringenin and eriodyctiol, as defined herein. To obtain such a working solution, a concentrate containing the components (a) and (b) according to the invention can be dissolved in, i.e., diluted by, a suitable amount of an additive, such as, for example, water, alcohol(s) or polyol(s), or mixtures of water, alcohol(s) and/or polyol(s). In such working solutions, it is considered that the relative amounts of the one or more compound according to general formula (Ia) or (Ib) and of the one or more compound selected from naringenin and eriodyctiol disclosed above for the concentrates according to the invention, remain the same when the working solution is prepared from the concentrate by dilution. As noted above, optionally, additional quantities of the one or more compound selected from naringenin and eriodyctiol may be added.

The compositions according to embodiments of the invention, either in the form of a concentrate or as a working solution, can be added to cosmetic and/or pharmaceutical preparations, for example, as is known from the use of glycerol monoalkyl ethers. In other embodiments, compositions according to embodiments of the invention may be used in technical products which are intended to be provided with glycerol monoalkyl ethers and in which peroxides are undesired, e.g., preparations comprising compounds which contain dyes or perfumes or which are unsaturated or sensitive to oxidation. Such preparations or technical products may include, for example, deodorants, skincare products, sunscreens, baby products, cosmetics, aftershaves, disinfectants, antiseptics, washing lotions, hair treatment compositions.

In some embodiments, the compositions according to the invention, whether added as concentrates or working solutions, are used in the preparations such as those examples above, such that the corresponding cosmetic, pharmaceutical or technical preparation contains from about 0.05 to about 5 wt%, or in another embodiment, from about 0.1 to about 1 wt%, or in another embodiment, from about 0.2 to about 0.6 wt%, or, in other embodiments 0.3 wt% or 0.5%, of the glycerol monoalkyl ether of the general formula (Ia) or (Ib) as defined herein. It is noted that, as the purity of the compound of formula (Ia) or (Ib) is increased, e.g., from 94% pure up to 98% pure, prior to distillation, a somewhat higher level of the antioxidant compound selected from naringenin and eriodyctiol may be required, since it appears that higher purity compound according to general formula (Ia) or (Ib) needs greater stabilization.

The foregoing example products may be prepared by simple mixing of the composition of the present invention together with the other components of the products.

## EXAMPLES

### Example 1

To demonstrate the antioxidant capabilities of the present invention, mixtures of 3-[(2-ethylhexyl)oxy]-1,2-propanediol, a compound having a general formula (Ia) as defined herein, which is marketed under the trade name SASKINE50™ by SACHEM, Inc., Austin, Texas, with naringenin, are prepared. The thus-prepared compositions are subjected to stability testing, under which formation of formaldehyde and peroxides and determined as indicators of stability, in which higher amounts of formaldehyde and/or peroxides are deemed to show lower stability of the mixture. As a first comparative example, a sample of 3-[(2-ethylhexyl)oxy]-1,2-propanediol, a compound having a general formula (Ia) as defined herein, is tested with no added antioxidant. As a second comparative example, a sample of commercially available 3-[(2-ethylhexyl)oxy]-1,2-propanediol, a compound having a general formula (Ia) as defined herein, but stabilized with synthetic alpha-tocopherol, an antioxidant disclosed and claimed in U.S. Patent No. 6,956,062, is tested. This sample is obtained commercially from Schulke GmbH. The tests for all samples determine the formaldehyde and peroxide contents of the test sample when stored at room temperature (RT), for periods of 0, 2, 4, 6, 8, 10 and 12 months. The 0 month sample is the freshly prepared composition.

The table below shows the components of the mixtures, and the graphs in FIG. 1 and 2 show the results of the

tests, for formaldehyde and peroxide formation, respectively.

| COMPOUND OF FORMULA (I) | SOURCE | ANTIOXIDANT | ANTIOXIDANT AMOUNT |
|---|---|---|---|
| EHOPD SASKINE™ 50 | SACHEM | None | None |
| EHOPD SASKINE™ 50 | SACHEM | Narigenin | 500 ppm |
| EHOPD SASKINE™ 50 | SACHEM | Narigenin | 1000 ppm |
| EHOPD SENSIVA® | SCHULKE | Synthetic alpha-tocopherol | 500 ppm |
| (The example with an amount of 1000 ppm antioxidant being not according to the invention) | | | |

As evident from Figs. 1 and 2, the naringenin-containing EHOPD, in accordance with the present invention, provides excellent antioxidant performance compared to both no antioxidant and the synthetic alpha-tocopherol antioxidant of the prior art. Applicant considers that naringenin is representative of the entire class of flavonoids disclosed and claimed in the present application.

Example 2

A further set of examples further demonstrates the efficacy of the present invention in providing antioxidant activity for 3-[(2-ethylhexyl)oxy]-1,2-propanediol, a compound having a general formula (Ia) as defined herein, which is marketed under the trade name SASKINE50™ by SACHEM, Inc., Austin, Texas. In this example, SACHEM's SASKINE50™ is used as basis for the stability testing of glycerol alkyl ethers within the scope of the present invention. A pre-defined blend containing SASKINE50™ and a number of the anti-oxidants compounds in accordance with the present invention are prepared, each containing 725 ppm of the antioxidant compound in 99.6% pure SASKINE50™ (3-[(2-ethylhexyl)oxy]-1,2-propanediol). The blend is analyzed via gas chromatography (GC) prior to stability testing. Following the analysis result, the blend is placed under atmospheric pressure and under an inert gas into individual sample bottles as not to disturb the testing conditions during sampling. The test conditions are 50°C for one month. During the month, the prepared samples are re-analyzed via gas chromatography at intervals of two weeks, for the SASKINE50™ (3-[(2-ethylhexyl)oxy]-1,2-propanediol) assay (loss of the compound) and for formation of volatile by-product. These test conditions are considered to simulate a shelf-life of approximately one year at room temperature.

Both the SASKINE50™ (3-[(2-ethylhexyl)oxy]-1,2-propanediol) and the volatile breakdown products are analyzed by GC. Breakdown products measured are those eluting prior to the SASKINE50™ (3-[(2-ethylhexyl)oxy]-1,2-propanediol). The samples are dissolved in isopropyl alcohol at a concentration of 10% for the GC analysis. The following GC conditions are used:

| | | |
|---|---|---|
| Column: | | |
| Material: | fused silica | WCOT |
| Length: | | 30 m |
| Internal diameter: | | 0.32 mm |
| Stationary phase: | | DB1701 |
| Film thickness: | | 1 μm |
| Gas regulation: | Carrier gas: | hydrogen |
| | Make-up gas: | helium |
| Temperatures: | Detector: | 300 °C |
| | Injector: | 275 °C |
| | Start temperature: | 115 °C |
| Column: | | |
| | Warm up speed | 1: 8 °C/minute |
| | End temperature: | 210 °C |
| | Warm up speed | 2: 15 °C/minute |
| | End temperature: | 275 °C |
| Detection: | Type detector: | FID |

FIG. 3 is a graph showing the loss of SASKINE50™ (3-[(2-ethylhexyl)oxy]-1,2-propanediol) on storage at the

elevated temperature of 50°C with the following compounds: naringenin, naringin, hesperitin, eriodictyol, quercetin, diosmetin, rhamnetin, norinhydrate and naringin hydrate (only naringenin and eriodyctiol are according to the invention). As shown in the graph in FIG. 3, when no antioxidant is included with the SASKINE50™ (3-[(2-ethylhexyl)oxy]-1,2-propanediol), a significant loss of shown during the test period, whereas when the antioxidant compounds in accordance with the present invention are added, there is almost no loss with all but two of the antioxidant compounds. It is noted that the antioxidant compounds naringin and naringin hydrate performed less well than did the other antioxidant compounds, so that these may be less favored in the present invention.

FIG. 4 is a graph showing the increase in volatile degradation products formed on storage of SASKINE50™ (3-[(2-ethylhexyl)oxy]-1,2-propanediol) at the elevated temperature of 50°C with the same antioxidant compounds as in the test shown in FIG. 3. It is noted that the low boiling compounds are reported as the sum of all detectable compounds which have a lower boiling point, i.e., that elute from the GC column prior to, compared to SASKINE50™ (3-[(2-ethylhexyl)oxy]-1,2-propanediol). As shown in FIG. 4, when no antioxidant is added to the SASKINE50™ (3-[(2-ethylhexyl)oxy]-1,2-propanediol), a significant amount of breakdown products are observed after both 2 weeks and 4 weeks at 50°C. By contrast, significantly smaller amounts of breakdown products are found when using the antioxidants according to the present invention. When the antioxidant compounds in accordance with the present invention are added, there are almost no breakdown products formed with all but two of the antioxidant compounds. It is noted that the antioxidant compounds naringin and naringin hydrate performed less well than did the other antioxidant compounds, so that these may be less favored in the present invention.

Example 3

A further set of examples further demonstrates the efficacy of the present invention in providing antioxidant activity for 3-[(n-octyl)oxy]-1,2-propanediol, a compound having a general formula (Ia) as defined herein, which is marketed under the trade name SASKINE80™ by SACHEM, Inc., Austin, Texas. In this example, SACHEM's SASKINE80™ is used as basis for the stability testing of one glycerol alkyl ether within the scope of the present invention, i.e., naringenin. A pre-defined blend containing SASKINE80™ and this anti-oxidant compound in accordance with the present invention are prepared, the blend containing 725 ppm of the naringenin in 99.6% pure SASKINE80™ (3-[(n-octyl)oxy]-1,2-propanediol). The blend is analyzed via gas chromatography (GC) prior to stability testing. Following the analysis result, the blend is placed under atmospheric pressure and under an inert gas into individual sample bottles as not to disturb the testing conditions during sampling. The test conditions are 50°C for two weeks and four weeks. During this time, the prepared samples are re-analyzed via gas chromatography at intervals of two weeks, for the SASKINE80™ (3-[(n-octyl)oxy]-1,2-propanediol) assay (loss of the compound) and for formation of volatile by-product. These test conditions are considered to simulate a shelf-life of approximately one year at room temperature.

Both the SASKINE80™ (3-[(n-octyl)oxy]-1,2-propanediol) and the volatile breakdown products are analyzed by GC, as described above for Example 2. FIG. 5 shows the results of stability testing of this blend. FIG. 5a shows the SASKINE80™ assay decrease over the two week and four week times at 50°C for SASKINE80™ alone and the blend of SASKINE80™ with naringenin. As illustrated in FIG. 5a, without the antioxidant, the SASKINE80™ assay decrease is about 0.40%, while with the naringenin, there is little or no assay loss for the SASKINE80™. FIG. 5b shows the increase in low boiling compounds over time at 50°C for SASKINE80™ alone and the blend of SASKINE80™ with naringenin. As illustrated in FIG. 5b, without the antioxidant, a notable increase in formation of low boiling compounds is observed after both two and four weeks, while with the antioxidant present, there is little or no increase in low boiling compounds over the same time periods at 50°C.

Example 4

As shown in Figs. 1 and 2, in the tests in Example 1, even at zero time, there is a measurable, non-zero amount of both peroxide and formaldehyde present in the SASKINE50™. In Example 1, the antioxidant was added subsequent to the manufacture of the SASKINE50™, raising the question of whether formation of the small amounts of peroxide and formaldehyde could be avoided by addition of the antioxidant immediately upon manufacture of the SASKINE50™. Thus, for this Example 4, samples of SASKINE50™ are combined with the antioxidant naringenin immediately upon manufacture, and were tested then (zero time) and three months later. The results are shown in FIG. 6. As shown in FIG. 6a, when the antioxidant is added immediately upon manufacture, the formation of peroxide is inhibited at both zero time and at three months, when stored at room temperature. As shown in FIG. 6b, the same amount of formaldehyde is present at zero time, as that shown at zero time in FIG. 2. However, as shown in FIG. 6b, at three months' time at room temperature, the formaldehyde content has not increased in this Example 4, while in the example shown in FIG. 2, there was an increase in formaldehyde for the same mixture of SASKINE50™ and naringenin when the naringenin is added subsequent to the manufacture of the SASKINE50™. Thus, it is demonstrated that addition of the antioxidant at the time the SASKINE50™ is manufactured has a significant benefit.

Example 5

As discussed in the background, the purpose of the glyceryl ether compounds such as SASKINE50™ and SASKINE80™ is as a preservative and antimicrobial, especially in cosmetics and pharmaceutical preparations. To test the SASKINE50™ and SASKINE80™, minimum inhibitory concentrations are determined for SASKINE50™ alone, SASKINE50™ with naringenin, SASKINE80™ alone, and SASKINE80™ with naringenin, and, for comparison, sensiva® SC 50, which contains 3-[(2-ethylhexyl)oxy]-1,2-propanediol and synthetic alpha-tocopherol, which is commercially available from Schülke & Mayr Benelux B.V., 2032 HA-Haarlem, Netherlands. The determination of the minimal inhibitory concentration (MIC) is carried out according to the standard procedure described below.

The purpose of the MIC study is to find a minimum concentration of the test item which will enable to inhibit *in vitro* the growth of a microbial strain. The MIC characterizes the bacteriostatic or fungistatic effect of a product.

In the MIC test, the product is diluted according to a geometric progression of reason 2 over a range of at least 5 dilutions starting from the maximum concentration to be tested specified by the customer. In each tube of the dilution range, a cultured medium double concentered is included with the strains to be tested. The density of the strains tested are around 106 CFU/ml for bacteria, 105 CFU/ml for yeasts and 104 CFU/ml for molds. Culture medium is Mueller Hinton for bacteria, and Sabouraud for yeast and molds. The incubation for inoculated tubes is at 32.5°C $\pm$ 2.5°C for 18-24h for bacteria, and at 30°C $\pm$ 2.5°C for 48h for yeasts and molds. The tubes showing turbidity related to the growth of the microorganisms are noted. The tubes having no turbidity are then transferred in order to count the remaining microorganisms. The seeded plates are incubated 3 to 5 days in the above-mentioned conditions respectively for bacteria and yeast and molds. Colonies counting on plates is used to calculate the number of CFU (Colony Forming Units) per gram or per ml of product. The MIC corresponds to the concentration of the first tube that does not show microbial turbidity. The CMB corresponds to the concentration of the first tube for which a decrease of 99.99% (4 Log reduction) is obtained for bacteria and of 99.90% (3 Log reduction) for yeasts and molds.

The results are shown in the table in FIG. 7. As shown in FIG. 7, in some cases addition of the stabilizer in ethylhexylglyceryl ether (SASKINE50™) to be more effective in anti-microbial growth compared both to plain material and Sensiva®. As shown in FIG. 7, the n-octyl glyceryl ether (SASKINE80™) shows an even better performance.

Example 6

As noted herein, the inventive compositions are intended for use in cosmetic and pharmaceutical compositions. One important criteria for such products is that there be no skin irritation, when the compositions are applied to the skin of the person using the cosmetic or pharmaceutical composition. To test the compositions of the present invention, a non-animal test is conducted, which is an *in vitro* skin irritation study of the compositions on human reconstructed epidermis known as the SkinEthic model, OECD 439. The objective of the study is to evaluate the ability of a product to cause cutaneous irritation by a cytotoxicity test combined with measuring Interleukin 1-alpha (IL1-$\alpha$) concentrations on reconstructed human epidermis in vitro. After the pure product has been applied to the epidermis for 42 minutes and they have been incubated for 42 hours post-treatment, cell viability is determined by measuring mitochondrial succinate dehydrogenase activity in living cells. This enzyme converts MTT (3(4.5-dimethylthiazol-2-yl)-2,5-diphenyltetrazolium bromide) into formazan blue crystals. A spectrophotometer is used to read the results after the crystals have been dissolved. The measured absorbances are proportional to the number of living cells. In addition, release of inflammation (IL1-$\alpha$) mediators is measured by colorimeter if cell viability is over 50%. This trial is conducted according to the protocol proposed by ECVAM on April 27th 2007 and the OECD 439. Cytotoxicity: absorbance is measured three times at 540nm. The results are expressed as a viability percentage compared to a negative control:

$$\%\ \text{viability} = \text{Sample absorption} \times 100 / \text{Negative control absorption}$$

IL1-$\alpha$ titration: IL1-$\alpha$ concentrations in the culture media are evaluated using the instructions on the titration kit and are expressed as pg/ml.

The test product is considered to be irritant for skin:

- if viability after a 42 minutes treatment and 42 hours incubation is less than 50%,
- if viability after a 42 minutes treatment and 42 hours incubation is greater than 50%, and the concentration of IL1-$\alpha$ is greater than 50 pg/ml.

The test product is considered to be non irritant for skin:

- if viability after a 42 minutes treatment and 42 hours incubation is greater than 50%, and the concentration of IL1-

$\alpha$ released is less than or equal to 50 pg/ml.

The results of the skin irritation test are shown in FIG. 8. As shown in FIG. 8, the skin irritation tests show that adding naringenin to SASKINE50™, in accordance with the present invention, changes the classification in this test from an irritant of category 1 or 2, to classification as a non-irritant. This is a key factor showing that the present invention provides an unexpected result when used as described herein and tested according to this protocol.

An advantage of the present invention obtains from the fact that naringenin is isolated from citrus sources, such as orange and grapefruit peel, making it easy to trace back its origin. Compositions in accordance with embodiments of the present invention may provide one or more of the following benefits: toxicologically acceptable; readily tolerated by the skin when applied topically; stable; largely and preferably completely odorless; inexpensive to prepare; easy to formulate and not detrimental to final products.

In one embodiment, the present invention provides compositions which comprise one or more glycerol monoalkyl ethers (compound of general formula (Ia) or (Ib)) and the one or more compound selected from naringenin and eriodyctiol, which are storage-stable for a long period under practical conditions, e.g., when stored at room temperature. In one embodiment, the composition is storage-stable up to 60 months, and in another embodiment, for a period ranging from 12 to 36 months. The compositions in accordance with the present invention should be protected from decomposition, in particular, should be protected from the development of high peroxide numbers, when the composition is tested according to testing procedures standard in the cosmetics and/or pharmaceutical industries.

## Claims

**1.** A composition comprising:

a. a glycerol alkyl ether of the general formula (Ia) or (Ib):

$$R\text{-}O\text{-}CH_2\text{-}CHOH\text{-}CH_2OH \qquad (Ia)$$

or

$$H\text{-}O\text{-}CHz\text{-}CH(OR)\text{-}CHzOH \qquad (Ib)$$

wherein, in general formula (Ia) or (Ib), R is a $C_3$-$C_{18}$ alkyl or alkenyl group, in which the alkyl or alkenyl group is branched or unbranched, unsubstituted or substituted by one or more hydroxyl, one or more $C_1$-$_{C4}$ alkoxy groups, or both one or more hydroxyl and one or more $C_1$-$_{C4}$ alkoxy groups, and the $C_3$-$C_{18}$ alkyl group, whether branched or unbranched, unsubstituted or substituted, is optionally interrupted by up to four oxygen atoms, or R is a $C_6$-$C_{10}$ aromatic hydrocarbon, unsubstituted or substituted by one or more hydroxyl, one or more $C_1$-$C_4$alkoxy groups, or both one or more hydroxyl and one or more $C_1$-$_{C4}$ alkoxy groups, and

b. one or more compound selected from naringenin and eriodyctiol,

wherein the composition contains from 300 ppm to 900 ppm of the one or more compound selected from naringenin and eriodyctiol, based on the content of the glycerol alkyl ether.

**2.** The composition of claim 1 wherein, in the general formula (Ia) or (Ib), R is a branched or unbranched $C_4$-$C_{12}$ alkyl group.

**3.** The composition of claim 1 wherein, in the general formula (Ia) or (Ib), R is 2-ethylhexyl.

**4.** The composition of claim 1 wherein, in the general formula (Ia) or (Ib), R is n-octyl.

**5.** The composition of claim 1 wherein, in the general formula (Ia) or (Ib), R is 2-octyl.

**6.** The composition of claim 1 wherein, in the general formula (Ia) or (Ib), R is a branched or unbranched $C_4$-$C_{12}$ alkyl group and the one or more compound selected from naringenin and eriodyctiol is naringenin.

**7.** The composition of claim 1 wherein either R is 2-ethylhexyl or R is n-octyl or R is 2-octyl, and the one or more compound selected from naringenin and eriodictyol is naringenin.

**8.** The composition of any one of claims 1-7 wherein the compound of general formula (Ia) or (Ib) is provided to the

composition at a purity of at least 95%, prior to addition of the one or more compound selected from naringenin and eriodyctiol.

**9.** A cosmetic or pharmaceutical composition comprising the composition of any of claims 1-8.

**10.** The composition of any preceding claim further comprising one or more additive selected from water, ethanol, propylene glycol.

## Patentansprüche

**1.** Zusammensetzung, umfassend:

a. einen Glycerolalkylether der allgemeinen Formel (Ia) oder (Ib):

$$R\text{-}O\text{-}CH_2\text{-}CHOH\text{-}CH_2OH \qquad (Ia)$$

oder

$$H\text{-}O\text{-}CH_2\text{-}CH(OR)\text{-}CH_2OH \qquad (Ib)$$

wobei in allgemeiner Formel (Ia) oder (Ib) R eine $C_3$-$C_{18}$-Alkyl- oder Alkenylgruppe ist, bei der die Alkyl- oder Alkenylgruppe verzweigt oder unverzweigt, unsubstituiert oder substituiert durch ein oder mehrere Hydroxyle, eine oder mehrere $C_1$-$C_4$-Alkoxygruppen oder sowohl ein oder mehrere Hydroxyle und eine oder mehrere $C_1$-$C_4$-Alkoxygruppen ist, und wobei die $C_3$-$C_{18}$-Alkylgruppe, ob verzweigt oder unverzweigt, unsubstituiert oder substituiert, optional unterbrochen ist durch bis zu vier Sauerstoffatome, oder wobei R ein aromatischer $C_6$-$C_{10}$-Kohlenwasserstoff ist, unsubstituiert oder substituiert durch ein oder mehrere Hydroxyle, eine oder mehrere $C_1$-$C_4$-Alkoxygruppen oder sowohl ein oder mehrere Hydroxyle und eine oder mehrere $C_1$-$C_4$-Alkoxygruppen, und

b. eine oder mehrere Verbindungen, ausgewählt aus Naringenin und Eriodyctiol,

wobei die Zusammensetzung 300 ppm bis 900 ppm der einen oder mehreren Verbindungen, ausgewählt aus Naringenin und Eriodyctiol, basierend auf dem Gehalt des Glycerolalkylethers, enthält.

**2.** Zusammensetzung nach Anspruch 1, wobei in der allgemeinen Formel (Ia) oder (Ib) R eine verzweigte oder unverzweigte $C_4$-$C_{12}$-Alkylgruppe ist.

**3.** Zusammensetzung nach Anspruch 1, wobei in der allgemeinen Formel (Ia) oder (Ib) R 2-Ethylhexyl ist.

**4.** Zusammensetzung nach Anspruch 1, wobei in der allgemeinen Formel (Ia) oder (Ib) R n-Octyl ist.

**5.** Zusammensetzung nach Anspruch 1, wobei in der allgemeinen Formel (Ia) oder (Ib) R 2-Octyl ist.

**6.** Zusammensetzung nach Anspruch 1, wobei in der allgemeinen Formel (Ia) oder (Ib) R eine verzweigte oder unverzweigte $C_4$-$C_{12}$-Alkylgruppe ist und die eine oder mehrere Verbindung ausgewählt aus Naringenin und Eriodyctiol Naringenin ist.

**7.** Zusammensetzung nach Anspruch 1, wobei R 2-Ethylhexyl ist oder R n-Octyl ist oder R 2-Octyl ist, und wobei die eine oder mehrere Verbindung ausgewählt aus Naringenin und Eriodyctiol Naringenin ist.

**8.** Zusammensetzung nach einem der Ansprüche 1 bis 7, wobei die Verbindung der allgemeinen Formel (Ia) oder (Ib) der Zusammensetzung in einer Reinheit von wenigstens 95% bereitgestellt wird, vor der Zugabe der einen oder mehreren Verbindung ausgewählt aus Naringenin und Eriodyctiol.

**9.** Kosmetische oder pharmazeutische Zusammensetzung, die die Zusammensetzung nach einem der Ansprüche 1 bis 8 umfasst.

**10.** Zusammensetzung nach einem vorangehenden Anspruch, weiter umfassend ein oder mehrere Additive, ausgewählt

aus Wasser, Ethanol, Propylenglykol.

**Revendications**

1. Composition comprenant :

   a. un éther alkylique de glycérol de la formule générale (Ia) ou (Ib)

   $$R\text{-}O\text{-}CH_2\text{-}CHOH\text{-}CH_2OH \qquad (Ia)$$

   ou

   $$H\text{-}O\text{-}CH_2\text{-}CH(OR)\text{-}CH_2\text{-}OH \qquad (Ib)$$

   dans laquelle, dans la formule générale (Ia) ou (Ib), R représente un groupe alkyle ou alcényle en $C_3$ à $C_{18}$, dans lequel le groupe alkyle ou alcényle est ramifié ou non ramifié, non substitué ou substitué par un ou plusieurs hydroxyle(s), un ou plusieurs groupe(s) alcoxy en $C_1$ à $C_4$, ou à la fois un ou plusieurs hydroxyle(s) et un ou plusieurs groupe(s) alcoxy en $C_1$ à $C_4$, et le groupe alkyle en $C_3$ à $C_{18}$, qu'il soit ramifié ou non ramifié, non substitué ou substitué, est éventuellement interrompu par jusqu'à quatre atomes d'oxygène, ou R représente un hydrocarbure aromatique en $C_6$ à $C_{10}$, non substitué ou substitué par un ou plusieurs hydroxyle(s), un ou plusieurs groupe(s) alcoxy en $C_1$ à $C_4$, ou à la fois un ou plusieurs hydroxyle(s) et un ou plusieurs groupe(s) alcoxy en $C_1$ à $C_4$, et
   b. un ou plusieurs composé(s) sélectionné(s) parmi la naringénine et l'ériodyctiol,
   dans laquelle la composition contient d'environ 300 ppm à environ 900 ppm d'un ou de plusieurs composés sélectionné(s) parmi la naringénine et l'ériodyctiol, sur la base de la teneur de l'éther alkylique de glycérol.

2. Composition selon la revendication 1, dans laquelle, dans la formule générale (Ia) ou (Ib), R représente un groupe alkyle en $C_4$ à $C_{12}$ ramifié ou non ramifié.

3. Composition selon la revendication 1, dans laquelle dans la formule générale (Ia) ou (Ib), R représente le 2-éthylhexyle.

4. Composition selon la revendication 1, dans laquelle dans la formule générale (Ia) ou (Ib), R représente le n-octyle.

5. Composition selon la revendication 1, dans laquelle, dans la formule générale (Ia) ou (Ib), R représente le 2-octyle.

6. Composition selon la revendication 1 dans laquelle, dans la formule générale (Ia) ou (Ib), R représente un groupe alkyle en $C_4$ à $C_{12}$ ramifié ou non ramifié et l'un ou plusieurs composé(s) sélectionnés parmi la naringénine et l'ériodyctiol, le choix étant la naringénine.

7. Composition selon la revendication 1 dans laquelle soit R représente le 2-éthylhexyle, soit R représente le n-octyle, soit R représente le 2-octyle, et l'un ou plusieurs composés sélectionné(s) parmi la naringénine et l'ériodyctiol, le choix étant la naringénine.

8. Composition selon l'une quelconque des revendications 1 à 7, dans laquelle le composé de formule générale (Ia) ou (Ib) est fourni à la composition à une pureté d'au moins 95 %, avant l'addition du ou des composés sélectionné(s) parmi la naringénine et l'ériodyctiol.

9. Composition cosmétique ou pharmaceutique comprenant la composition selon l'une quelconque des revendications 1 à 8.

10. Composition selon l'une quelconque des revendications précédentes comprenant en outre un ou plusieurs additifs sélectionnés parmi l'eau, l'éthanol, le propylène glycol.

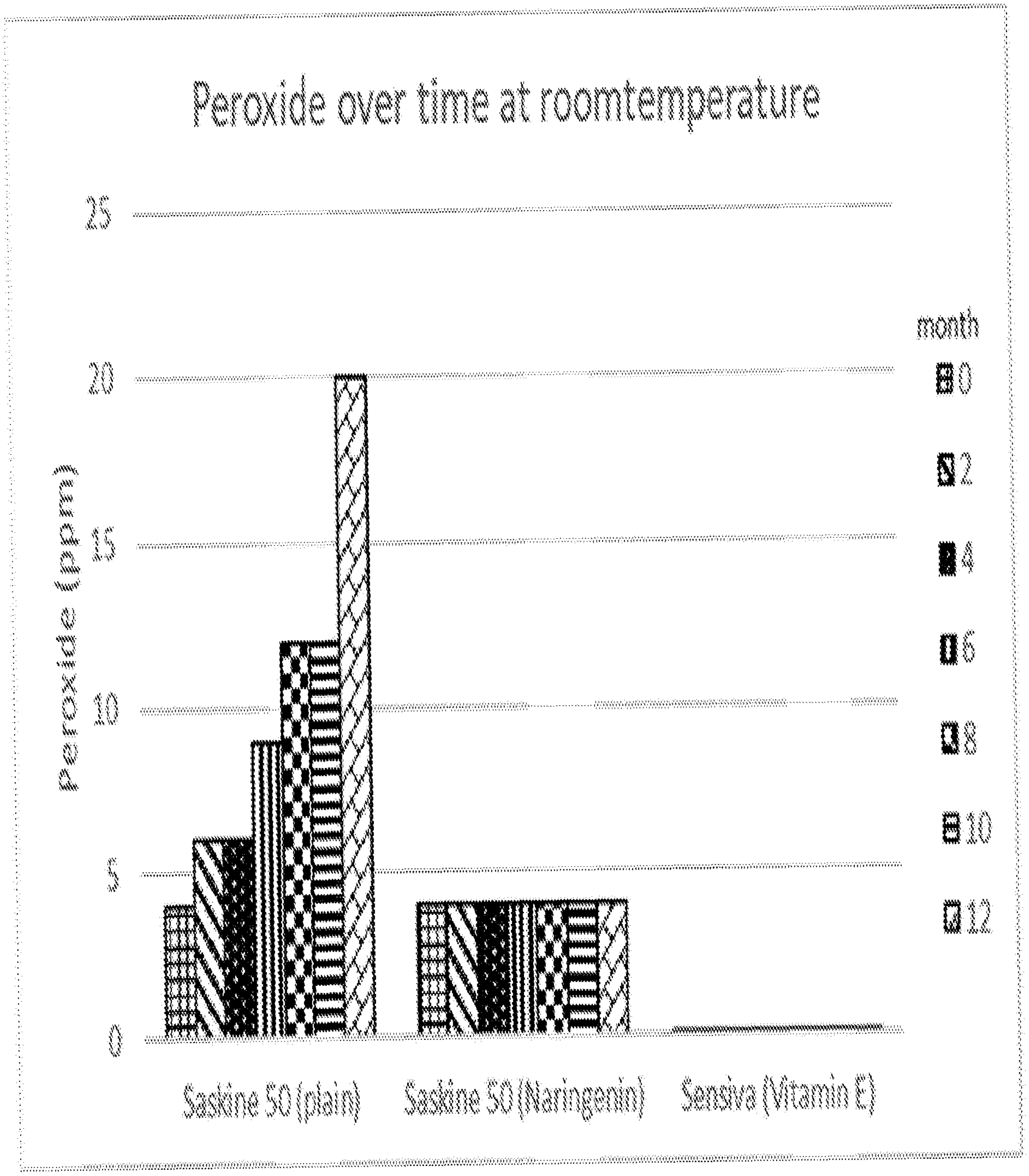
Peroxide over time at roomtemperature
peroxide (ppm)
25
20
15
10
5
0
Saskine 50 (plain)
Saskine 50 (Naringenin)
Sensiva (Vitamin E)
month
0
2
4
6
8
10
12

FIG. 1

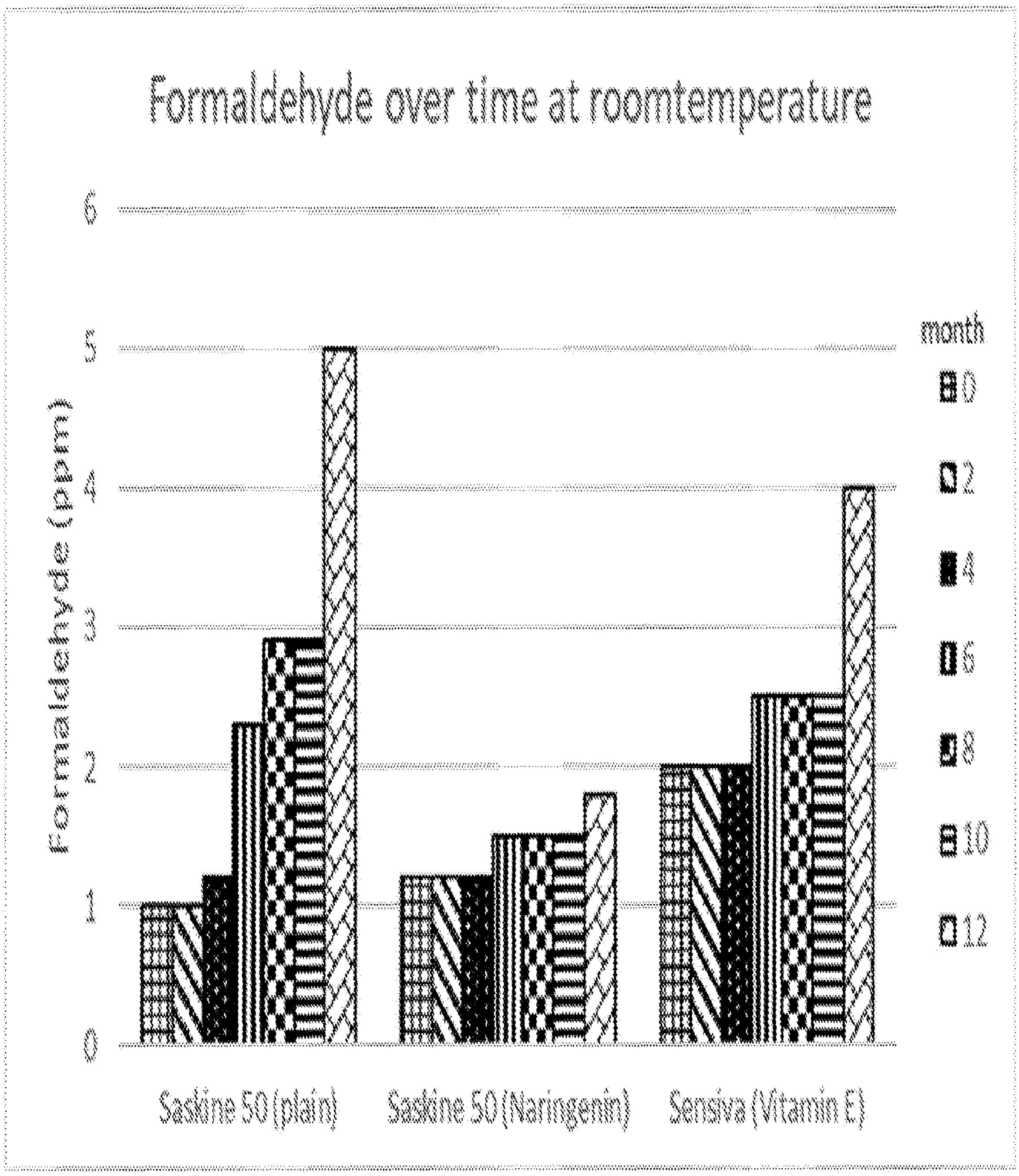
Formaldehyde over time at roomtemperature
Formaldehyde (ppm)
6
5
4
3
2
1
0
month
0
2
4
6
8
10
12
Saskine 50 (plain)
Saskine 50 (Naringenin)
Sensiva (Vitamin E)

FIG. 2

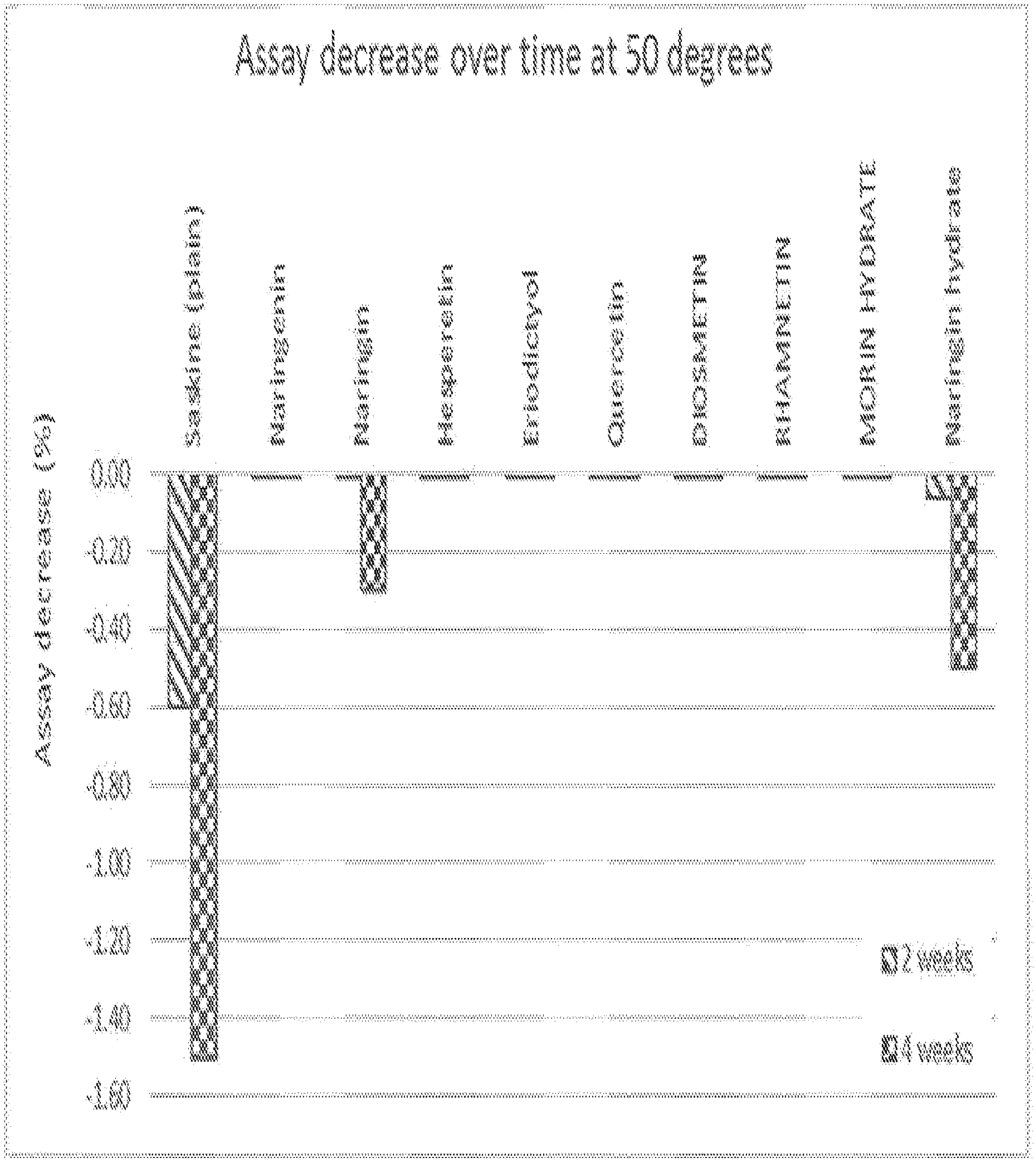
Assay decrease over time at 50 degrees
Assay decrease (%)
0.00
-0.20
-0.40
-0.60
-0.80
-1.00
-1.20
-1.40
-1.60
Saxkine (plain)
Naringenin
Naringin
Hesperetin
Eriodictyol
Quercetin
DIOSMETIN
RHAMNETIN
MORIN HYDRATE
Naringin hydrate
2 weeks
4 weeks

FIG. 3

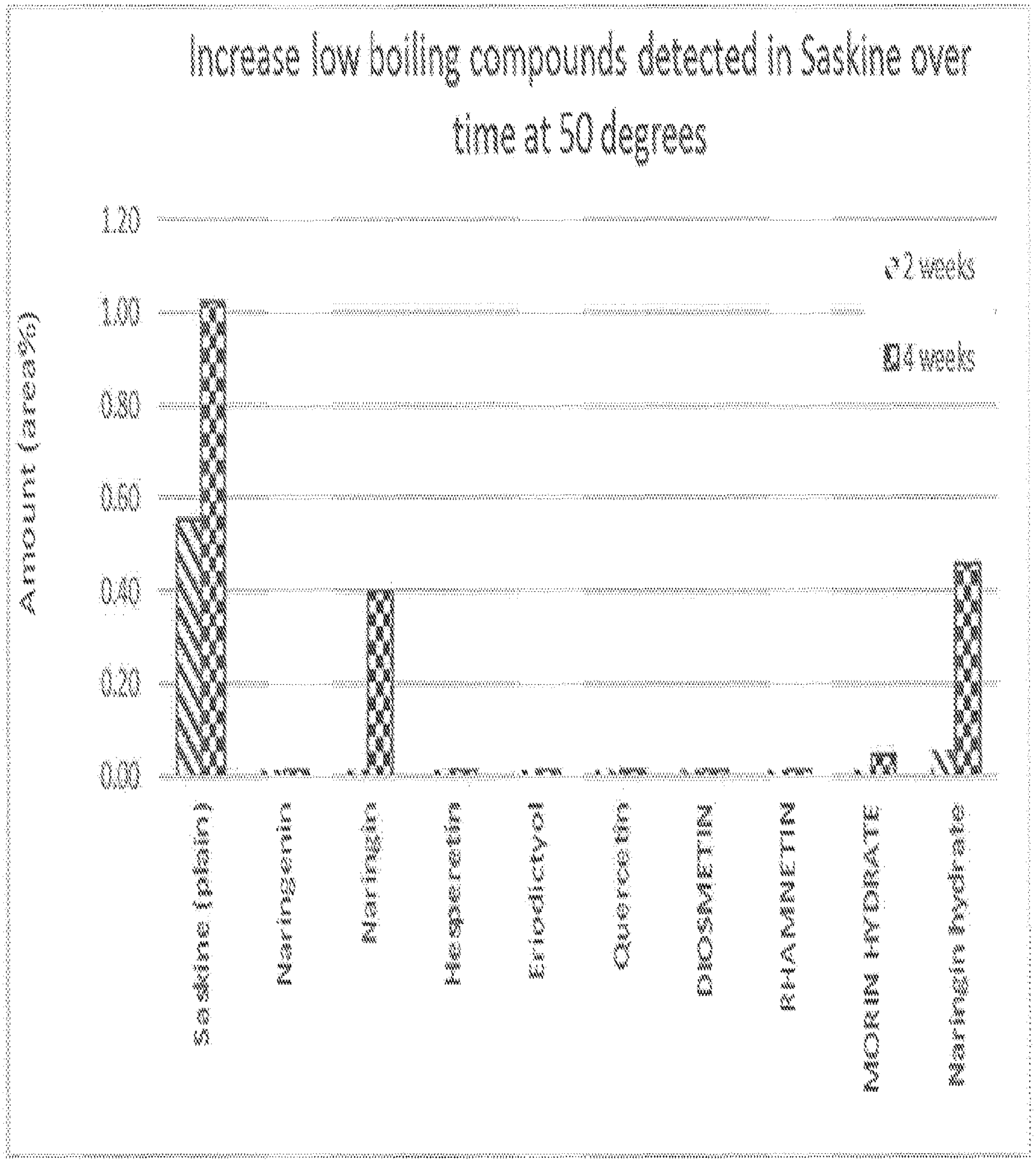

Increase low boiling compounds detected in Saskine over time at 50 degrees
Amount (area%)
1.20
1.00
0.80
0.60
0.40
0.20
0.00
2 weeks
4 weeks
Saskine (plain)
Naringenin
Naringin
Hesperetin
Eriodictyol
Quercetin
DIOSMETIN
RHAMNETIN
MORIN HYDRATE
Naringin hydrate


FIG. 4

FIG. 5a

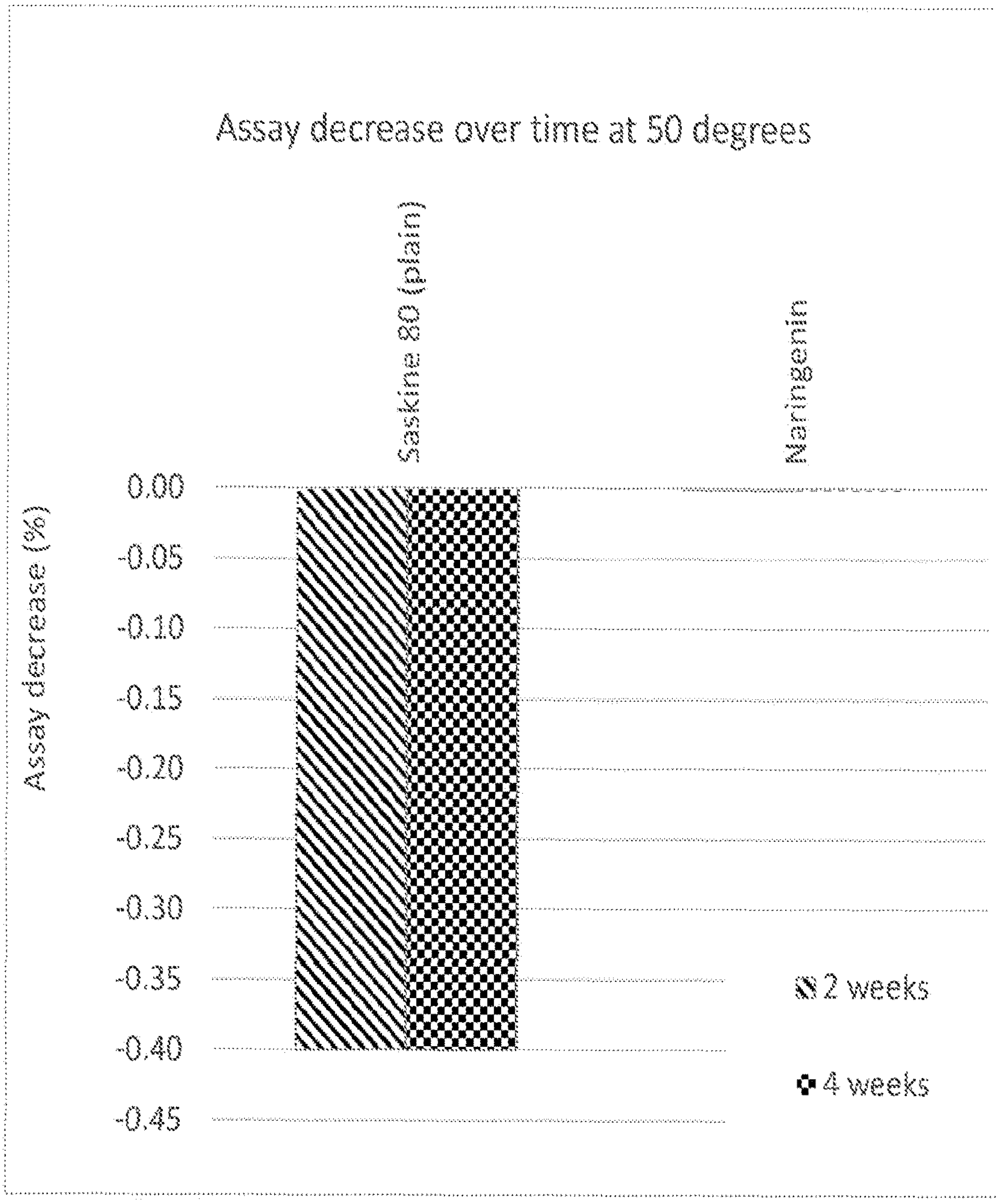
Assay decrease over time at 50 degrees
Saskine 80 (plain)
Naringenin
Assay decrease (%)
0.00
-0.05
-0.10
-0.15
-0.20
-0.25
-0.30
-0.35
-0.40
-0.45
2 weeks
4 weeks

FIG. 5b

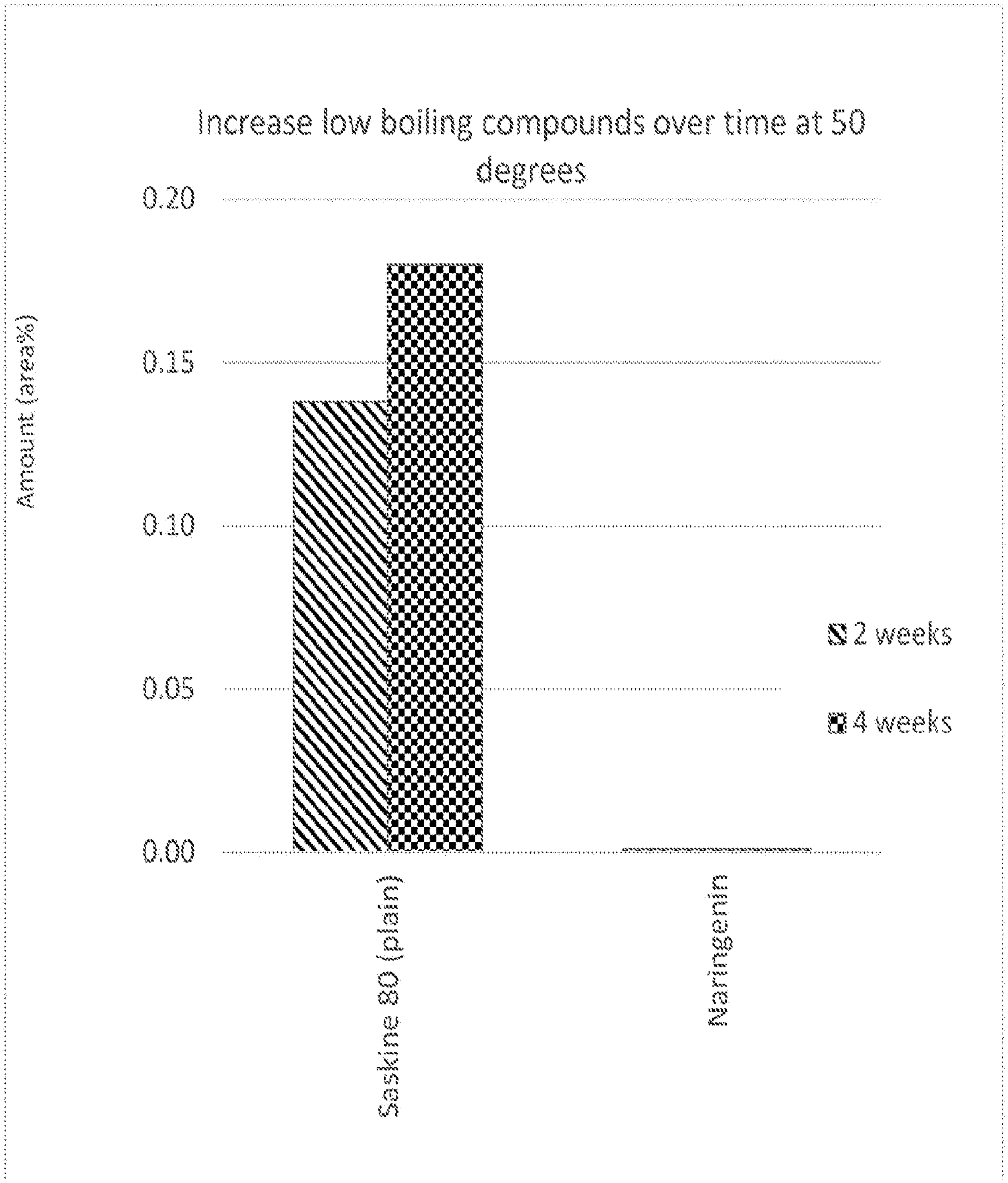
Increase low boiling compounds over time at 50 degrees
0.20
0.15
0.10
0.05
0.00
Amount (area%)
2 weeks
4 weeks
Saskine 80 (plain)
Naringenin

FIG. 6a

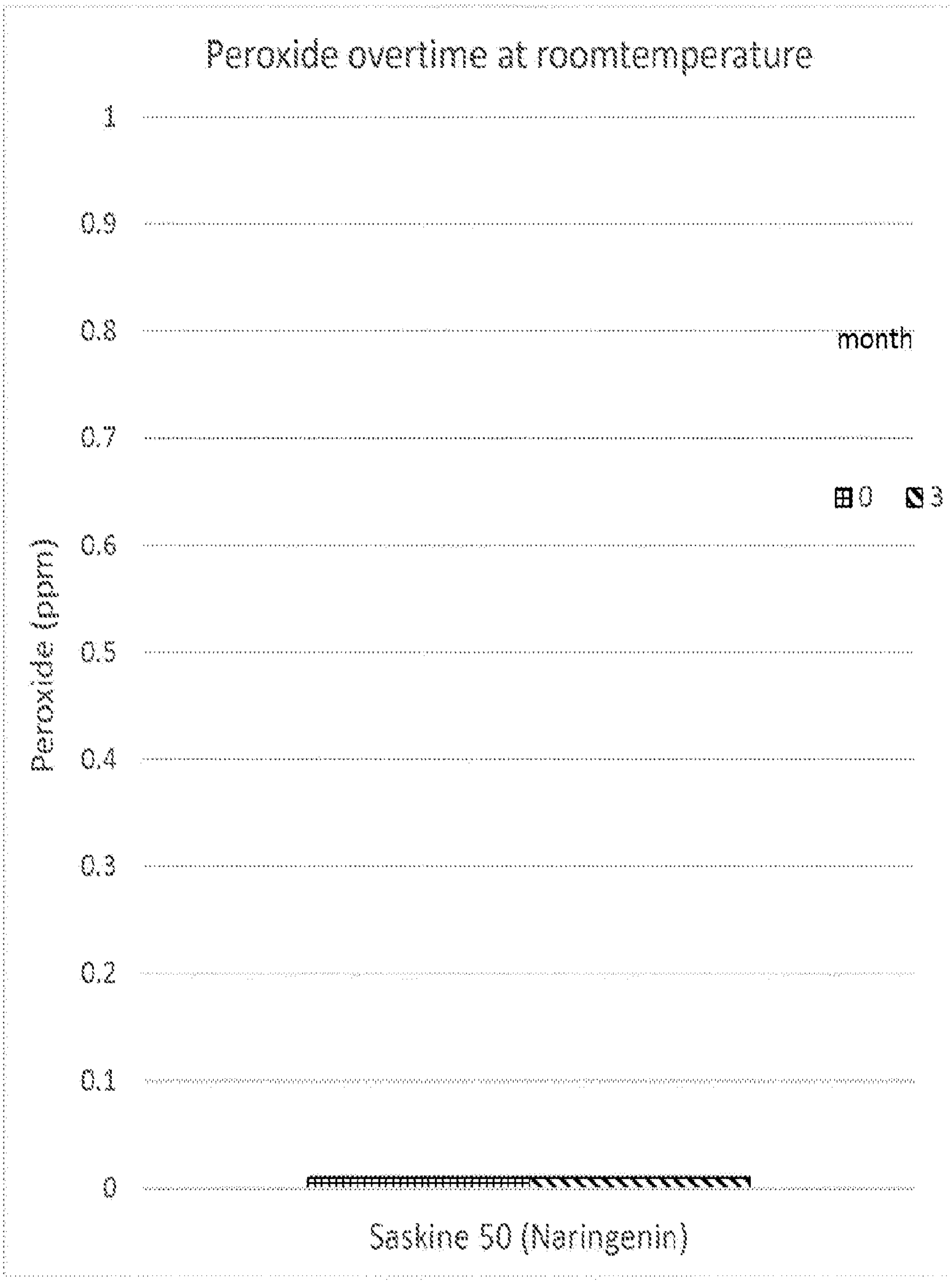
Peroxide overtime at roomtemperature
1
0.9
0.8
0.7
0.6
0.5
0.4
0.3
0.2
0.1
0
Peroxide (ppm)
month
0
3
Saskine 50 (Naringenin)

FIG. 6b

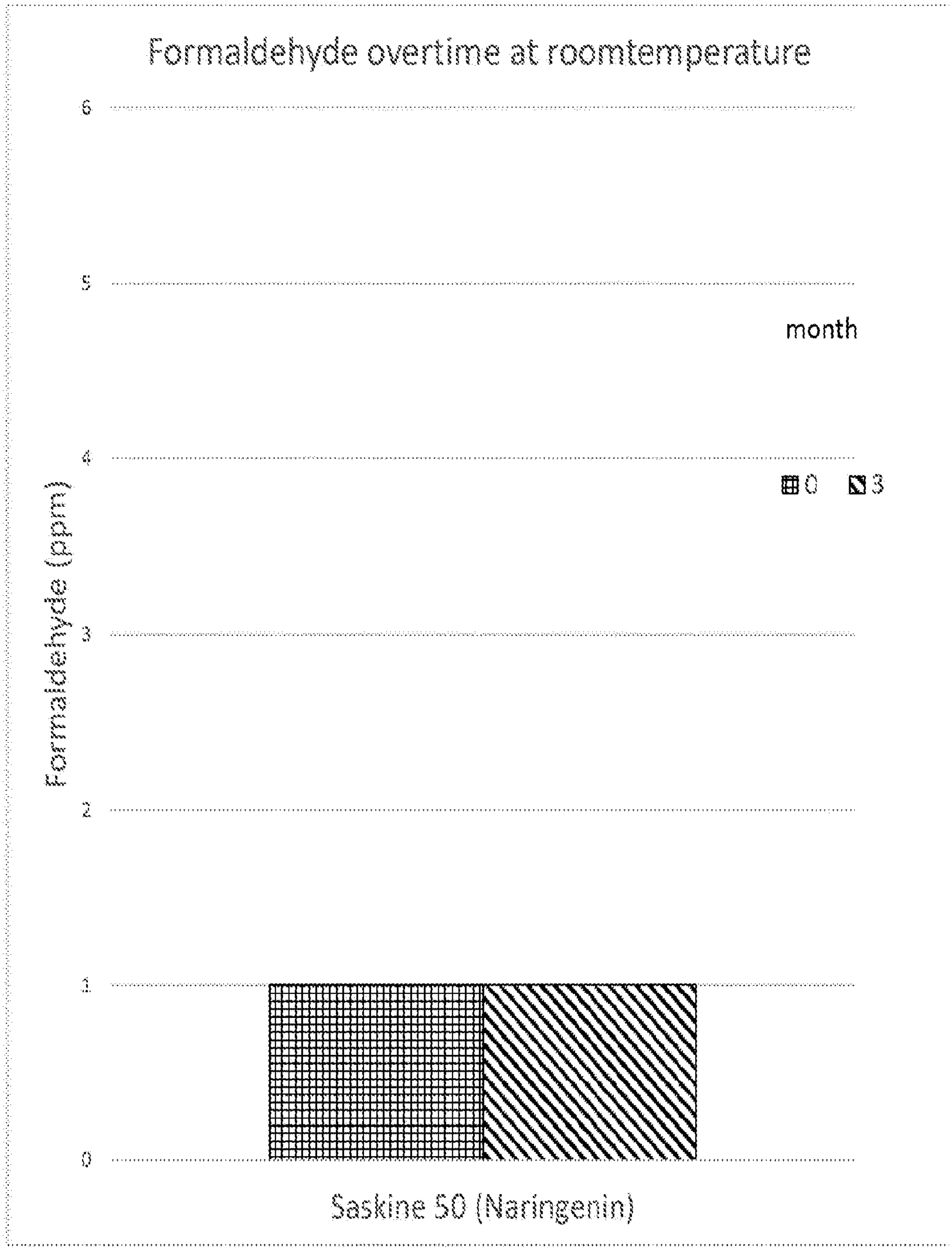
Formaldehyde overtime at roomtemperature
Formaldehyde (ppm)
6
5
4
3
2
1
0
month
0
3
Saskine 50 (Naringenin)

FIG. 7

| Microbiological MIC in ppm at pH 7.0 | Saskine™50 | Saskine™50 + Naringenin | Saskine™80 | Saskine™80 + Naringenin | Sensiva SC 50 |
|---|---|---|---|---|---|
| *Staphylococcus aureus* DSM 799 | 1000 | 1000 | 1000 | 500 | 1000 |
| *Pseudomonas aeruginosa* DSM 1128 | 2500 | 2500 | 1500 | 1000 | 1500 |
| *Escherichia coli* DSM 682 | 2000 | 1500 | 1000 | 1000 | 2000 |
| *Enterobacter aerogenes* NCIMB 10102 | 2500 | 2500 | 1000 | 1000 | 2500 |
| *Klebsiella pneumoniae* DSM 786 | 500 | 500 | 1000 | 500 | 500 |
| *Burkholderia cepacia DSM 7288 ** | 1500 | 200 | 1000 | 1000 | 1500 |
| *Enterobacter gergoviae DSM 9245 ** | >10000 | >10000 | >10000 | >10000 | >10000 |
| *Citrobacter freundii NCIMB 12203* | 2500 | 2000 | 2000 | 1000 | 1500 |
| *Candida albicans* DSM 1386 | 1000 | 1000 | 500 | 500 | 1000 |
| *Aspergillus brasiliensis* DSM 1988 | 500 | 500 | 250 | 250 | 1000 |
| *Penicillium minioluteum IMI 178519* | 250 | 500 | 250 | 250 | 500 |
| *Aspergillus terreus IMI 45543* | 500 | 1000 | 250 | 250 | 1000 |

| Microbiological MIC in ppm at pH 7.0 | Saskine$^{tm}$50 | Saskine$^{tm}$50 + Naringenin | Saskine$^{tm}$80 | Saskine$^{tm}$80 + Naringenin | Sensiva SC 50 |
|---|---|---|---|---|---|
| *Fusarium solani IMI 322444* | 2500 | 1000 | 500 | 500 | 1000 |
| *Penicillium funicolosum ATCC 11797* | NT | NT | NT | NT | NT |
| *Saccharomyces cerevisiae DSM 70449* | 500 | 1000 | 500 | 500 | 1000 |
| *Candida parapsilosis DSM 5784 ** | 1500 | 1500 | 500 | 500 | 1000 |

FIG. 8

# RESULTS

***In vitro* skin irritation study of a test item on human reconstructed epidermis (SkinEthic model) according to the OECD guideline n°439**

**Protocol identification:** 6.32_S version 03A
**Start of experimentation:** Week 34
**End of experimentation:** Week 40
**Study Director:** Michèle PARMANTIER

| Sponsor | Test Facility |
|---|---|
| SACHEM EUROPE B.V<br>Van Voordenpark 15<br>5301 KP ZALTBOMMEL<br>NETHERLANDS | IDEA Lab<br>Technopôle Montesquieu<br>5, rue Jacques Monod<br>CS 60077<br>33652 MARTILLAC Cedex<br>FRANCE |

| Test item | Study number | Dilution | Mean Viability Assay 1 | Mean Viability Assay 2 | Final Result |
|---|---|---|---|---|---|
| EHG-ST - REF : SASKINE TM 50 | 6.32_S -40226-ID-17/06601 | pure | 48.2% | 66.4% | Non Irritant* |
| EHG - REF : SASKINE TM 50 | 6.32_S -40226-ID-17/06599 | pure | 54.8% | 34.4% | Category 1 or 2** |

* EHG-ST - REF : SASKINE TM 50:
In assay 1, the mean viability of the epidermises was 50 ± 5%, the results had to be confirmed because in the equivocal zone.
In assay 2, the mean viability of the epidermises was 66.4%.
In conclusion the test item is considered as non irritant.

** EHG - REF : SASKINE TM 50 :
In assay 1, the mean viability of the epidermis was 50 ± 5%, the results had to be confirmed because in the equivocal zone.
In assay 2, the mean viability of the epidermis was 34.4%.
In conclusion the test item must be classified Category 1 or 2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6956062 B
- CN 105997754 A
- DE 102006062566 A1
- FR 2923159 A1
- EP 1537849 A1
- DE 19063345 A1